# EUROPEAN PATENT APPLICATION

(11) **EP 3 967 678 A1**
(43) Date of publication of application: **16.03.2022**
(21) Application number: 20735463.0
(22) Date of filing: 08.05.2020
(51) Int. Cl.: C07C 233/56, C01G 56/00, C07C 231/02, C07C 231/12, C07C 231/24, C07C 233/02, C22B 3/00, C22B 7/00, C22B 59/00

(54) **PROCESS FOR THE SYNTHESIS OF THE IONIC LIQUID TETRAOCTYLAMMONIUM DI(2-ETHYLHEXYL)-OXAMATE (IL-5), PRODUCT OBTAINED AND ITS USE IN SELECTIVE METAL EXTRACTION**

(30) Priority: 09.05.2019 PT 2019115507
(71) Applicant: Instituto Superior Técnico, 1049-001 Lisboa (PT)
(72) Inventor: ARRIEGAS ESTEVÃO CORREIA LEAL, João Paulo, LISBOA (PT); DA CUNHA OLIVEIRA FIGUEIRA CARRETAS, José Manuel, LISBOA (PT); MARIA CRUZ, Adelaide, LISBOA (PT); DE JESUS MARIA, Leonor Maria, LISBOA (PT); RAMOS BATISTA MONTEIRO, Bernardo, LISBOA (PT)
(74) Representative: Pereira da Cruz, Joao
(86) International application number: PCT/PT2020/050019
(87) International publication number: WO 2020/226522

(57) **Abstract**

The present invention falls within the area of the synthesis of ionic liquids, namely it concerns a process of synthesis of the ionic liquid tetraoctylammonium di(2-ethylhexyl)-oxamate (IL-5) with a high degree of purity and its use in the extraction and selective separation of metals, namely lanthanides. Thus, it is the object of the present invention, a process for the synthesis of a pure ionic liquid using in its constitution only the elements carbon, hydrogen, oxygen and nitrogen (CHON), assuming itself as a "green" alternative in the recovery of metals, thus reducing the environmental impact in the way they are recovered, as well as the guarantee of a more efficient extraction of these metals.

## Description

### STATE OF THE ART

The present invention falls within the area of the synthesis of ionic liquids, namely it concerns the process of synthesis of an ionic liquid composed only of elements such as carbon, hydrogen, oxygen and nitrogen and its use in the selective extraction of metals, namely lanthanides.

Ionic liquids are characterized for being constituted by a bulky organic cation and an organic or inorganic anion, characterized by a low vapor pressure, low flammability and high thermal stability, and are commonly used in a huge variety of industrial processes as solvents, lubricants, catalysts and other uses.

Among the various industrial processes in which they can be applied, its use as a means of extracting and recovering metals from secondary sources and for environmental remediation, represents one of the relevant applications of ionic liquids. More specifically, its use in the recovery of metals such as rare earths (RE), in which lanthanides are included, is one of the promising possibilities worldwide.

Rare earths, scandium, yttrium and lanthanides, are fundamental elements in today's industry, with increasing applications in the components of numerous products, from micro-electronics to wind power generators, including hybrid cars. The importance of these compounds is related to the fact that world production is dominated by China (97%), which in 2012 introduced export quotas. China does the mining of the RE but has also specialized in extraction processes of the ores, in the separation of the different RE from concentrates, in the production of permanent magnets based on RE and luminescent materials from purified RE.

The European Commission has considered rare earth as a high-risk raw material [Critical Raw Materials for EU, European Commission, 2010], and has been accompanied in these concerns by the United States [Critical Materials Strategy, US Department of Energy, December 2011] and Japan. On March 13, 2012, the United States, Europe and Japan filed a formal complaint to the World Trade Organization (WTO) regarding restrictions imposed by China on the export of RE. Five of the REs - dysprosium, terbium, europium, neodymium and yttrium - are considered the most critical as they have a very significant importance in the processes of producing clean energy while presenting a high risk of supply in both the short and medium term.

Thus, there is a need to increase and diversify sources of obtaining rare earths, namely lanthanides, including recovery and recycling from end-of-life products. The recovery of these compounds is still at very low levels (~ 1%) due to inefficient collection, technological problems, lack of incentives and little research in this regard.

In order to face this emerging need for recovery and/or recycling of metals through secondary sources, it is essential to develop ionic liquids with more effective properties, namely with regard to their extraction capacity and greater selectivity in relation to a variety of metals, that is, the objective is to be able to synthesize ionic liquids that allow to selectively extract different metals in order to obtain more pure metals.

From the point of view of its use, the present invention represents a more efficient use and a more efficient recovery of lanthanides through a high selectivity of extraction among the various lanthanides.

These properties were obtained through a suitable combination of a bulky organic cation and an anion, with the ability to coordinate with lanthanides, in order to form an ionic liquid immiscible in water, but with the ability to extract metals from an aqueous phase to an organic phase.

Additionally, in any application, the ionic liquids to be used must have a positive contribution to the environment, that is, the environmental impact resulting from their use must be kept to a minimum. The overwhelming majority of ionic liquids currently in use contain in their composition elements such as fluorine (F), chlorine (Cl) or sulfur (S), which, upon decomposition or elimination of the ionic liquids, lead to the formation of acidic, chlorinated or fluorinated compounds, such as dioxins, which have a very negative effect from an environmental point of view.

The present invention, in addition to ensuring the synthesis of an ionic liquid efficient in the recovery of metals, does so through an ionic liquid containing only carbon (C), hydrogen (H), oxygen (O) and nitrogen (N) which ensures its low environmental impact since its decomposition or elimination does not lead to the formation of compounds harmful to the environment. At the same time, by extracting the metals from urban and industrial waste through the use of the ionic liquid, object of the present invention, the amount of metals that are deposited in landfills is also being reduced, so the present invention is also a strong contribution to the reduction of pollution in the environment.

Existing the need to recycle metals to supply a western market avid for these compounds, it is imperative to recover them through "green" solutions whose impact on the environment is as little as possible.

The ionic liquid IL-5, object of the present invention, and its use has an important value in society since its use in the recovery and selective separation of lanthanides has an important economic and financial effect as it makes their recovery and separation easier. By removing these metals from urban and industrial waste, the amount of metals deposited in landfills is also being reduced, so the present invention is also a strong contribution to the reduction of pollution in the environment. Often rare earths and in particular lanthanides are recovered in a mixture of the various elements. The similarity of their chemical properties makes their separation very difficult. The use of the IL-5 ionic liquid ensures the possibility of an effective separation of the lanthanides throughout the series.

In short, the process, the product and its use, object of the present invention, contribute to the state of the art with the following advantages:
- ionic liquid free of elements such as fluorine, chlorine and sulfur, which after degradation lead to the formation of compounds harmful to health and the environment;
- efficient use in the extraction of lanthanides;
- high selectivity in the extraction of lanthanides;
- simple and economical process of synthesis.

### SUMMARY OF THE INVENTION

Therefore, the object of the present invention is the synthesis process of ionic liquid tetraoctylammonium di(2-ethylhexyl)-oxamate (IL-5) prepared from a solution of di(2-ethylhexyl)-methyl-oxamate, to which an aqueous solution of sodium hydroxide was added to obtain sodium di(2-ethylhexyl)-oxamate, which in turn is dissolved in an ether and stirred with an aqueous solution of tetraoctylammonium chloride, obtaining the ionic liquid tetraoctylammonium di(2-ethylhexyl)-oxamate (IL-5) as a yellowish viscous liquid after removal of the solvent from the organic phase by vacuum.

It is also an object of the present invention to obtain the ionic liquid tetraoctylammonium di(2-ethylhexyl)-oxamate (IL-5), with a high degree of purity, which being composed only of elements such as carbon, hydrogen, oxygen and nitrogen (CHON), can be considered a "green" alternative in the recovery and recycling of metals.

The ionic liquid tetraoctylammonium di(2-ethylhexyl)-oxamate (IL-5), obtained from the synthesis process referred in the present invention, is used in the recovery and recycling of metals, namely lanthanides. The use of this ionic liquid allows a more efficient extraction of the lanthanides and a greater selectivity during the extraction of the lanthanides.

### DESCRIPTION OF THE FIGURES

Figure 1 - representation of the schematic formula of the ionic liquid tetraoctylammonium di(2-ethylhexyl)-oxamate (IL-5).
Figure 2 - representation of the proton nuclear magnetic resonance spectrum, ¹H NMR, of the ionic liquid tetraoctylamonium di(2-ethylhexyl)-oxamate in CDCl₃.
Figure 3 - representation of the carbon 13 nuclear magnetic resonance spectrum, ¹³C NMR, of the ionic liquid tetraoctylammonium di(2-ethylhexyl)-oxamate in CDCl₃.
Figure 4 - representation of the absorption spectrum in the infrared region of the ionic liquid tetraoctylammonium di(2-ethylhexyl)-oxamate.
Figure 5 - graphical representation of the differential thermal analysis of the ionic liquid tetraoctylammonium di(2-ethylhexyl)-oxamate.
Figure 6 - graphical representation of the thermogravimetric analysis of the ionic liquid tetraoctylammonium di(2-ethylhexyl)-oxamate.
Figure 7 - graphical representation of the percentage of extraction of each lanthanide using the ionic liquid tetraoctylammonium di(2-ethylhexyl)-oxamate.
Figure 8 - graphical representation of the percentage of extraction of each lanthanide as a function of the variation in the mixing time using the ionic liquid tetraoctylammonium di(2-ethylhexyl)-oxamate.
Figure 9 - graphical representation of the percentage of extraction of each lanthanide as a function of pH variation using the ionic liquid tetraoctylammonium di(2-ethylhexyl)-oxamate.
Figure 10 - graphical representation of the percentage of extraction of each lanthanide as a function of the variation of the ionic strength of the medium using the ionic liquid tetraoctylamonium di(2-ethylhexyl)-oxamate.
Figure 11 - graphical representation of the percentage of extraction of each lanthanide as a function of the variation in the ratio between the ionic liquid and the existing metal using the ionic liquid tetraoctylammonium di(2-ethylhexyl)-oxamate.
Figure 12 - graphical representation of the percentage of retroextraction of each lanthanide to an aqueous phase using acidic solutions of HNO₃.

### DETAILED DESCRIPTION OF THE INVENTION

The synthesis process of the ionic liquid tetraoctylammonium di(2-ethylhexyl)-oxamate (IL-5) comprises the following steps:
a) preparation of di(2-ethylhexyl)-methyl-oxamate by reacting di(2-ethylhexyl)amine with methyl-oxalyl chloride;
b) addition of an aqueous solution of sodium hydroxide to di(2-ethylhexyl)-methyl-oxamate, obtained in step a), dissolved in a non-halogenated organic solvent with some polarity;
c) stirring the reaction mixture obtained in the previous step at a temperature between 20 and 30°C and subsequent removal of the solvent by vacuum, obtaining a solid residue;
d) dissolving the solid residue obtained in the previous step in a short chain ether;
e) simple filtration with filter paper of the solution obtained in the previous step and removal of the solvent from the filtrate by vacuum, obtaining sodium di(2-ethylhexyl)-oxamate as a white solid;
f) addition of an aqueous solution of tetraoctylammonium chloride to an ether solution of sodium di(2-ethylhexyl)oxamate prepared from the product obtained in the previous step;
g) stirring the solution;
h) separation of the aqueous phase from the organic phase;
i) washing the organic phase with water to completely remove salts and new separation of the two phases;
j) removal of the solvent from the organic phase under vacuum;
k) obtaining the ionic liquid tetraoctylammonium di(2-ethylhexyl)-oxamate (IL-5) in the form of a slightly yellowish viscous liquid.

Di(2-ethylhexyl)-methyl-oxamate is the starting compound for the synthesis of the IL-5 ionic liquid, object of the present invention. It is a reagent not commercially available in the chemical industry, so its preparation is carried out by reacting di(2-ethylhexyl)amine with methyl-oxalyl chloride as described on page 200 of the PhD thesis of Axel Braam, PhD thesis, Philips-Universitat Marburg, 2015.

In a preferred mode of the invention the solvent used in step b) is tetrahydrofuran, since through the use of this solvent a higher yield is obtained.

In another preferred mode of the invention the reaction mixture described in step c) is made at a room temperature between 20 and 30 °C for 5 days.

In another preferred mode of the invention, the ether used in step d) is diethyl ether since a higher yield is obtained through the use of this type of ether.

In step e) of the process described above, simple filtration with filter paper is performed in order to remove the non-soluble fraction and then the ether of the filtrate is removed by vacuum to obtain sodium di(2-ethylhexyl)-oxamate.

In another preferred mode of the invention the reaction mixture described in step g) is made at a room temperature between 20 and 30 °C for 4 hours.

From the point of view of its use, the present invention, presents a more efficient use and a more efficient recovery of the lanthanides through a great selectivity of extraction among the several lanthanides. This selectivity is due to the type of coordination that the anion of the ionic liquid establishes with the different metals.

### Example 1

Di(2-ethylhexyl)-methyl-oxamate was prepared as described on page 200 of the Doctoral thesis by Axel Braam, PhD thesis, Philips-Universitat Marburg, 2015. A sodium hydroxide solution (0.235 g; 59 mmol) in 7.5 ml of ultrapure Millipore water (ISO 3696) was slowly added to a solution of di(2-ethylhexyl)-methyl oxamate (1.542 g; 47 mmol) dissolved in tetrahydrofuran (7.5 ml). The reaction mixture was stirred at room temperature for 5 days. After stirring the reaction mixture, the solvent was removed under vacuum and a solid residue was obtained, which was dissolved in 15 ml of diethyl ether and filtered. The solvent was again removed under vacuum to obtain a white solid with a yield of 79% (1.35 g) compared to the initial di(2-ethylhexyl)-methyl-oxamate. Subsequently, tetraoctilammonium chloride (1.802 g, 36 mmol) dissolved in Millipore water (90 mL) was slowly added to a solution of sodium di(2-ethylhexyl)oxamate (1.253 g, 37 mmol) in diethyl ether (125 mL) and the previous solution was vigorously stirred for 4 hours at room temperature. After stirring, the aqueous phase was separated from the organic phase, washing the latter with Millipore ultra-pure water (1 × 50 mL). The organic solvent was removed under vacuum and the desired ionic liquid was obtained as a slightly yellowish viscous liquid with a yield of 84% (2.412 g) relative to the sodium salt used.

The ionic liquid tetraoctylammonium di(2-ethylhexyl)-oxamate (IL-5), object of the present invention obtained by the described process has the following molecular structure:

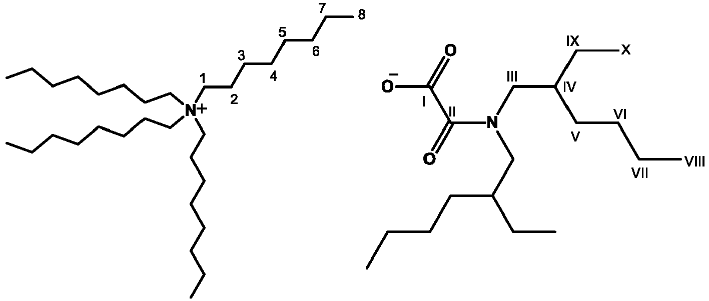

In addition, the ionic liquid IL-5 was characterized through various analytical techniques to ascertain its purity and verify some of its fundamental properties for different types of use.

The degree of purity of the IL-5 obtained allows to obtain non contaminated extracts and thus to clearly analyze its performance in the extraction of lanthanides.

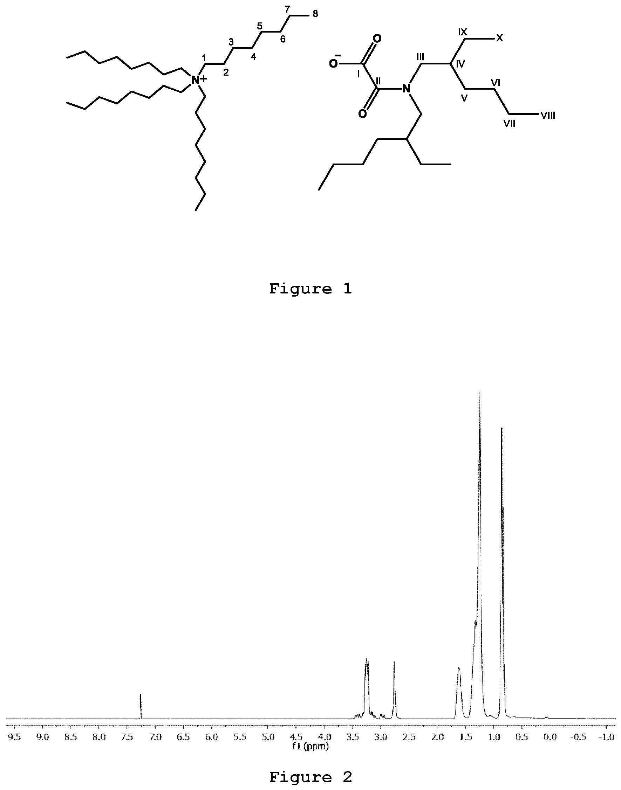

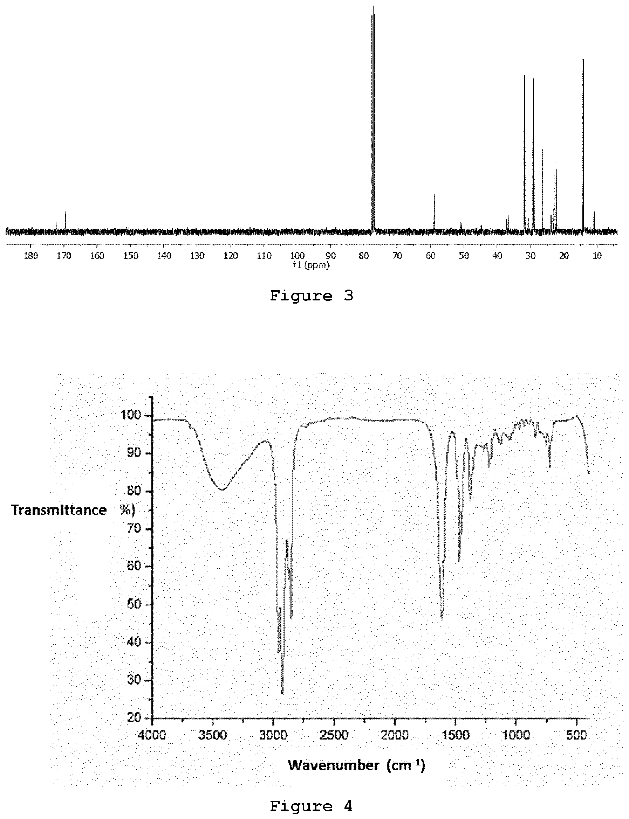

Proton (Figure 2) and carbon (Figure 3) nuclear magnetic resonance spectra were performed, of which the most relevant data are presented below: ¹H NMR (300 MHz, CDCl₃, δ ppm) : 3.48-2.92 (m, 4H + 8H, H^{III} from oxamate and H¹ from [N₈₈₈₈]⁺), 1.71-1.50 (m, 2H + 8H, H^{IV} from oxamate and H² from [N₈₈₈₈]⁺), (m, 16H + 40H, H^{V-VII,IX} from oxamate and H³⁻⁷ from [N₈₈₈₈]⁺), 0.76-0.93 (m, 12H + 12H, H^{VIII,X} from oxamate and H⁸ from [N₈₈₈₈]⁺); ¹³C NMR (75 MHz, CDCl₃, δ ppm); 172.29 (C^{I}), 169.52 (C^{II}), 58.91 (C¹), 50.95, 50.80, 44.89, 44.65 (C^{III}), 37.19, 37.13, 36.61, 36.52 (C^{IV}), 31.80 (C⁶), 30.78, 30.76, 30.72, 30.63 (C^{V}), 29.25, 29.14 (C^{4,5}), 29.10, 29.03, 28.99 (C^{IX}), 26.43 (C^{2,3}), 23.93, 23.80, 23.68, 23.22, 23.19, 23.16, 22.70, 22.23 (C⁷+ C^{VI,VII}), 14.27 14.25, 14.23 (C^{VIII}), 14.15 (C8), 11.21, 11.18, 10.90 (C^{x}) 10.85.

These data make it possible to identify the different magnetic resonances corresponding to the different hydrogen and carbon atoms in the ionic liquid, all of which are assigned according to the numbering in Figure 1.

Thus, from the analysis carried out to the proton and carbon nuclear magnetic resonance spectra, it appears that there are no other signals in the spectra, so that the IL-5 ionic liquid synthesized by the previously described process is obtained as a pure compound.

The infrared spectrum of the compound under study is also presented (Figure 4). This spectrum clearly identifies the functional groups existing in the ionic liquid and together with the nuclear magnetic resonance spectra presented before, prove the purity of the compound.

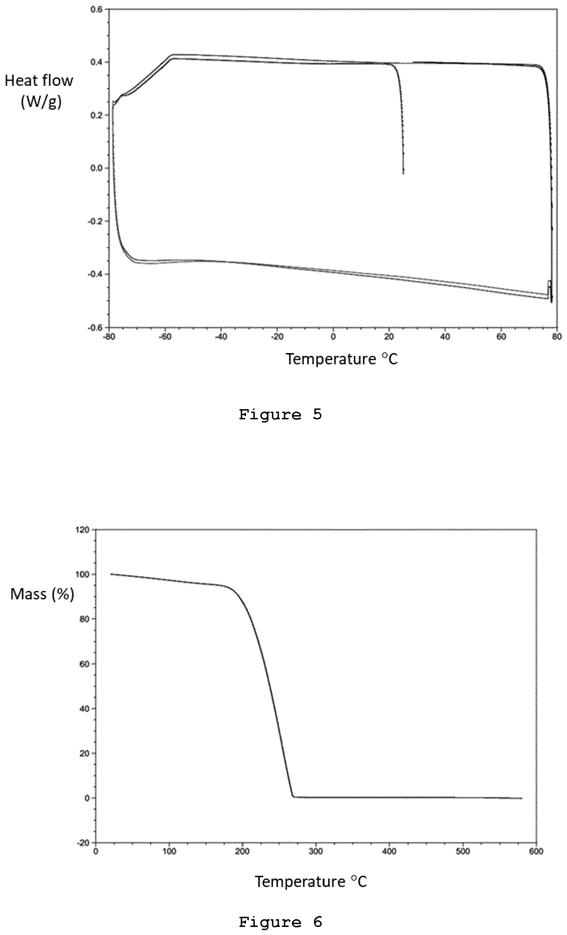

Differential thermal analysis (Figure 5) and thermogravimetric analysis (Figure 6) of the ionic liquid tetraoctylammonium di(2-ethylhexyl)-oxamate were also performed.

The first analysis also proves that the product is obtained pure by this synthesis process, since it does not contain in the studied temperature range (-80 °C to 80 °C) any other detectable compound nor does it show any phase change. The thermogravimetric analysis also shows that the compound is pure because its thermal decomposition does not leave any residue and in addition guarantees that at 270 °C the decomposition of the ionic liquid is total. These analyses ensure that its decomposition does not leave a residue, which together with the fact that its constitution contains only carbon, hydrogen, oxygen and nitrogen (CHON), guarantees that all ionic liquid is transformed by low temperature combustion into volatile compounds with low environmental impact.

The ionic liquid tetraoctylammonium di(2-ethylhexyl)-oxamate (IL-5), object of the present invention, obtained by the described process has favorable stereochemical characteristics for its use in the selective separation of metals and among these rare earths (lanthanides).

The following example exemplifies the use of the ionic liquid IL-5 in the selective separation of lanthanides.

### Example 2

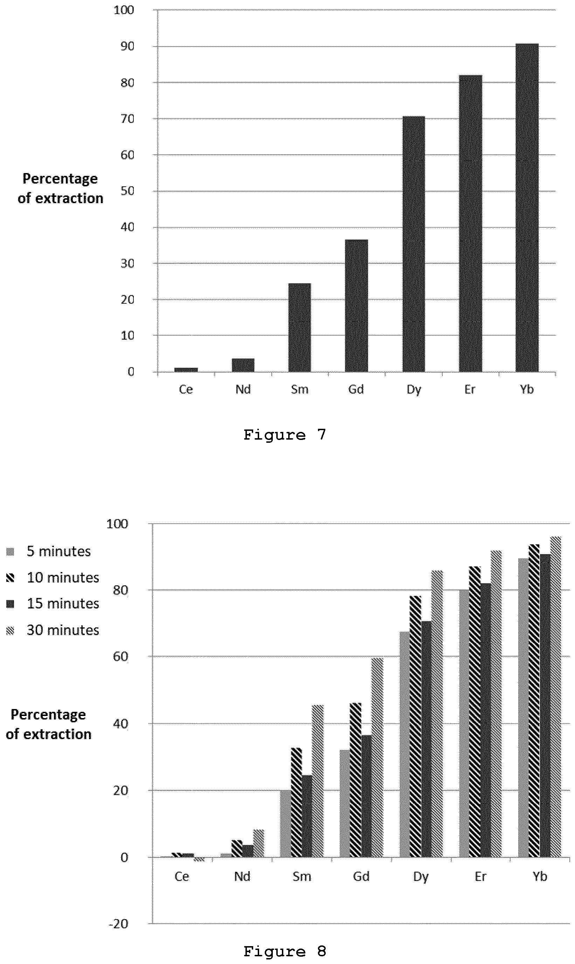

An aqueous solution was prepared containing several lanthanides with an approximate content of 100 ppm for each lanthanide and a pH of about 4. The initial concentration of each lanthanide in the solution was measured by inductively coupled plasma mass spectrometry (ICP-MS) as shown in table 1. Selective separation was carried out by adding 1 ml of a solution of toluene with 400×7 ppm of ionic liquid tetraoctylamonium di(2-ethylhexyl)-oxamate (IL5) to 1 ml of the solution of lanthanides previously prepared and stirred on a vortex mixer for 15 minutes, followed by centrifugation for an additional 5 minutes. At the end, the organic phase was separated from the aqueous phase and the final concentration of the various lanthanides in the aqueous phase was measured using ICP-MS, the percentage of extraction of each one of the lanthanides was calculated. The results are presented in Table 1 and illustrated in Figure 7. The studied lanthanides uniformly cover the entire series of them and therefore the conclusions drawn here can be generalized to all lanthanides.

**Table 1- Results of the percentage of extraction of each lanthanide according to the described process.**

| Ppm | Ce | Nd | Sm | Gd | Dy | Er | Yb |
|---|---|---|---|---|---|---|---|
| Initial value | 100.70 | 100.10 | 102.00 | 88.80 | 95.30 | 100.20 | 103.60 |
| Final value | 99.60 | 96.50 | 77.00 | 56.40 | 28.00 | 18.00 | 9.50 |
| % extraction | 1.09 | 3.60 | 24.51 | 36.49 | 70.62 | 82.04 | 90.83 |

The extraction efficiency was studied as described in example 2, varying the extraction contact time, that is, vortex agitation time, with agitation times of 5, 10, 15 and 30 minutes being studied. The analysis of the results obtained represented in Figure 8 shows that there is no significant difference in the percentage of extraction with this variable.

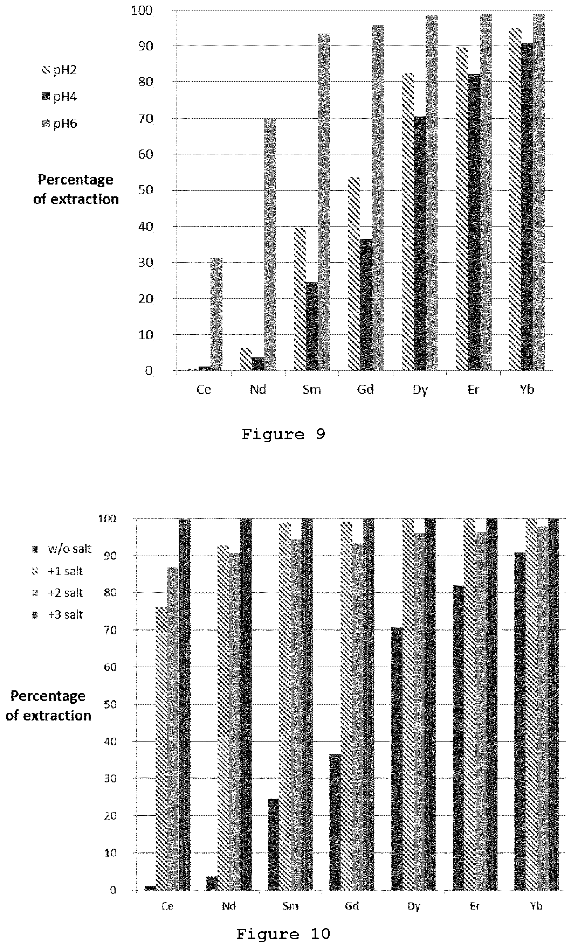

The efficiency of the extraction was studied as described in example 2, varying the value of the acidity of the medium (pH), having been studied solutions with a pH of 2, 4 and 6. The analysis of the results obtained represented in Figure 9 demonstrate that the extraction is most effective with a pH medium of 4.

The extraction efficiency was further studied as described in example 2, varying the ionic strength of the medium, with four aqueous solutions being studied, three of which with different amounts of sodium nitrate, which are: 0.0085 g (1×10⁻⁴ mols), 0.0425 g (5×10⁻⁴ mols) and 0.085 g (1×10⁻³ mols) .

The extraction efficiency was studied as described in example 2, varying the ratio between the ionic liquid and the existing metals, in the molar ratio ionic liquid/metal of 2:1 and 4:1.

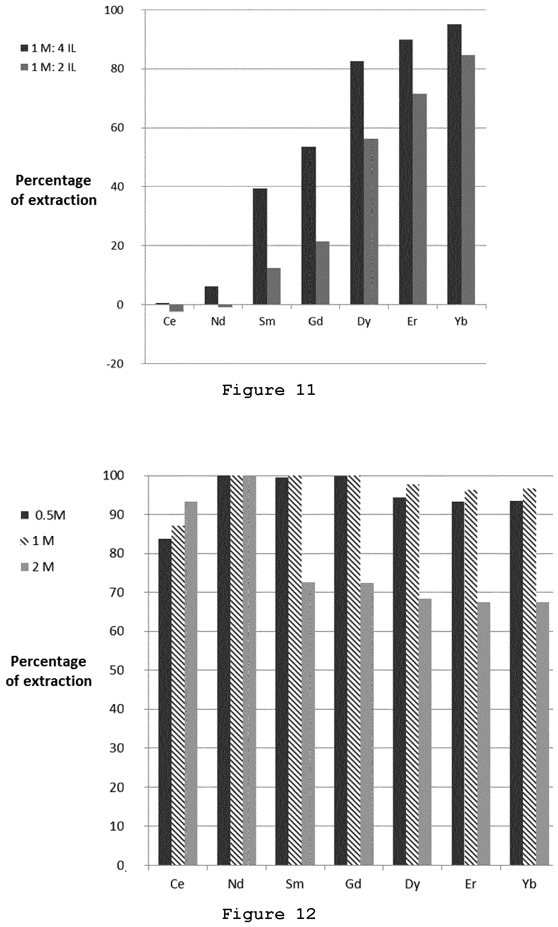

After a selective extraction to a non-aqueous phase containing the ionic liquid as described above, it is important to check whether it is possible to put the extracted metals back into the aqueous phase, thus completing the recovery cycle. For this purpose, an extraction of the toluene solution obtained in the process described in example 2 was carried out using nitric acid in three different concentrations: 0.5 M, 1 M and 2 M to see how the concentration influenced this retroextraction. The obtained results are shown in Figure 12.

## Claims

1. Process of synthesis of the ionic liquid tetraoctylammonium di(2-ethylhexyl)-oxamate (IL-5) **characterized by** comprising the following steps:
a) preparation of di(2-ethylhexyl)-methyl oxamate by reacting di(2-ethylhexyl)amine with methyl-oxalyl chloride;
b) adding an aqueous solution of sodium hydroxide to di (2-ethylhexyl)-methyl oxamate obtained in step a) dissolved in a non-halogenated organic solvent with some polarity;
c) stirring the solution obtained in the previous step at a temperature between 20 and 30 °C and removing the solvent by vacuum, obtaining a solid residue;
d) dissolving the residue obtained in the previous step in a short-chain ether;
e) simple filtration with filter paper of the solution obtained in the previous step, removal of the solvent from the filtrate by vacuum, obtaining sodium di(2-ethylhexyl)-oxamate as a white solid;
f) adding a solution of tetraoctylammonium chloride in water to a solution of sodium di(2-ethylhexyl)oxamate dissolved in an ether prepared from the product obtained in the previous step;
g) stirring the solution;
h) separation of the aqueous phase from the organic phase;
i) washing the organic phase with water;
j) removing the solvent from the organic phase under vacuum;
k) obtaining the ionic liquid tetraoctylammonium di(2-ethylhexyl)-oxamate (IL-5) as a slightly yellowish viscous liquid.

2. Process according to claim 1, **characterized by,** in step b), the solvent used is tetrahydrofuran.

3. Process according to claim 1, **characterized by,** in step c), the reaction mixture is made at a temperature between 20 and 30 °C for 5 days.

4. Process according to claim 1, **characterized by,** in step d), the ether is diethyl ether.

5. Process according to claim 1, **characterized by,** in step g), the reaction mixture is carried out at a temperature between 20 and 30 °C for 5 days.

6. Ionic liquid tetraoctylammonium di(2-ethylhexyl-oxamate (IL-5) obtained through the process described in claims 1 to 5, is **characterized by** having the molecular structure:

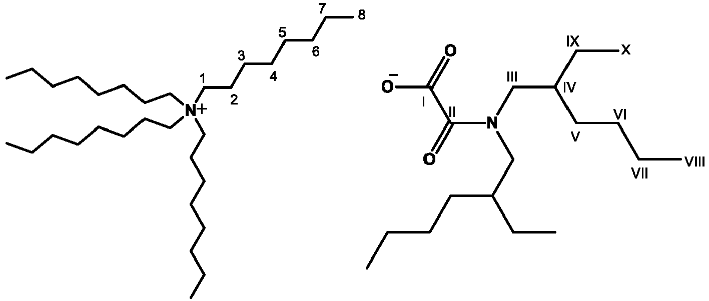

7. Ionic liquid tetraoctylammonium di(2-ethylhexyl)-oxamate (IL-5) ionic liquid according to claim 6, **characterized by** having a proton and carbon nuclear magnetic resonance spectrum of ¹H NMR (300 MHz, CDCl₃, δ ppm): 3.48-2.92 (m, 4H + 8H, H^{III} from oxamate and H¹ from [N₈₈₈₈]⁺), 1.71-1.50 (m, 2H + 8H, H^{IV} from oxamate and H² from [N₈₈₈₈]⁺), (m, 16H + 40H, H^{V-VII,IX} from oxamate and H³⁻⁷ from [N₈₈₈₈]⁺), 0.76-0.93 (m, 12H + 12H, H^{VIII,X} from oxamate and H⁸ from [N₈₈₈₈]⁺); ¹³C NMR (75 MHz, CDCl₃, δ ppm); 172.29 (C^{I}), 169.52 (C^{II}), 58.91 (C¹), 50.95, 50.80, 44.89, 44.65 (C^{III}), 37.19, 37.13, 36.61, 36.52 (C^{IV}), 31.80 (C⁶), 30.78, 30.76, 30.72, 30.63 (C^{V}), 29.25, 29.14 (C^{4,5}), 29.10, 29.03, 28.99 (C^{IX}), 26.43 (C^{2,3}), 23.93, 23.80, 23.68, 23.22, 23.19, 23.16, 22.70, 22.23 (C⁷+ C^{VI,VII}), 14.27 14.25, 14.23 (C^{VIII}), 14.15 (C⁸), 11.21, 11.18, 10.90 (C^{x}) 10.85.

8. Ionic liquid tetraoctylammonium di(2-ethylhexyl)-oxamate (IL-5) according to claims 6 and 7 **characterized by** its total thermal decomposition at 270 °C.

9. Use of the ionic liquid tetraoctylammonium di(2-ethylhexyl)-oxamate (IL-5) described in claims 6 to 8 in the selective extraction of lanthanides.
